# EUROPEAN PATENT SPECIFICATION

(11) **EP 1 278 513 B1**
(45) Date of publication and mention of the grant of the patent: **18.07.2007**
(21) Application number: 01930970.7
(22) Date of filing: 01.05.2001
(51) Int. Cl.: A61K 9/16, A61K 31/155, A61K 31/4439, A61K 9/50

(54) **A CORE FORMULATION**
KERNFORMULIERUNG
FORMULATION DE NOYAUX

(30) Priority: 01.05.2000 US 201567 P; 31.10.2000 US 702332
(43) Date of publication of application: 29.01.2003
(73) Proprietor: Aeropharm Technology, LLC, Dover, DE 19901 (US)
(72) Inventor: CUTIE, Anthony, J., Bridgewater, NJ 08807 (US); ADJEI, Akwete, L., Bridgewater, NJ 08807 (US)
(74) Representative: Findlay, Alice Rosemary
(86) International application number: PCT/US2001/013988
(87) International publication number: WO 2001/082867

(56) References cited:
- WO-A-00/27401
- WO-A-01/35940
- WO-A-01/35941
- WO-A-98/57634
- US-A- 4 166 800
- US-A- 5 496 557
- US-A- 5 916 584
- US-A- 6 011 049
- US-A- 6 099 862
- DEASY P B ET AL: "COMBINED DIPYRIDAMOLE AND ASPIRIN PELLET FORMULATION FOR IMPROVED ORAL DRUG DELIVERY. PART 1: DEVELOPMENT PHARMACEUTICS" JOURNAL OF MICROENCAPSULATION, TAYLOR AND FRANCIS INC. LONDON, GB, vol. 13, no. 4, 1 July 1996 (1996-07-01), pages 385-394, XP000589902 ISSN: 0265-2048

## Description

This application claims priority from U.S. provisional application Serial No. 60/201,567 filed May 1, 2000, which is incorporated herein by reference.

### BACKGROUND OF THE INVENTION

### Field of the Invention

This invention relates to a core formulation, and, more particularly, to a core formulation comprising a first layer comprising rosiglitazone, which covers at least a portion of a core comprising the biguanide, such as for example metformin (i.e., glucophage).

### Description of the Related Art

Metformin and rosiglitazone, or their salts such as the hydrochlorides, maleates, tartrates, etc., are two active ingredients of anti-diabetic drugs that are used to treat diabetic patients, e.g. human beings. These two active agents are administered orally to patients in need thereof in protocols calling for the single administration of either ingredient. Heretofore, there has not been revealed or hinted at combining both ingredients and certainly not a physically combined core formulation comprising both ingredients. The use of such a core formulation is advantageous to patients and prescribers because both medicaments are synergistic to each other in the body when used in the management of blood glucose control, i.e., diabetes.

### SUMMARY OF THE INVENTION

This invention relates to a core formulation, and, more particularly, to a core formulation comprising a first layer comprising rosiglitazone, which covers at least a portion of a core comprising a biguanide. A typical biguanide is metformin. It typically is used clinically as a pharmaceutically acceptable salt, preferably the hydrochloride salt. A commercial form of metformin hydrochloride is available as glucophage. Its chemical name is N,N-dimethylimidodicarbonimidic diamide hydrochloride. Metformin hydrochloride is a hydrochloride salt of metformin base, and as used herein, "metformin" means the base compound as well as its pharmaceutically acceptable salts. Metformin is used clinically to manage non-insulin deficient diabetes mellitus ("NIDDM"), particularly in patients who are not effectively treated with a sulfonylurea. While it is not chemically related to the sulfonylureas, it is routinely utilized in combination with a sulfonylurea, and has been shown to be synergistic in some cases. Other biguanides such as phenformin, buformin etc. can also be used. Additionally, in the treatment of a diabetic patient the metformin, for example, and the rosiglitazone are present in effective amounts to provide such treatment.

### DETAILED DESCRIPTION OF THE INVENTION

Metformin is an active ingredient for a commercially available drug employed to treat diabetes mellitus in a host or mammal, e.g. a human being, another animal. The typical daily effective dose for the oral treatment of a mammal, i.e., a human, ranges from 500 mg to 2550 mg. Typically, the dose is a single dose of 500 mg to 850 mg.

Rosiglitazone hydrochloride, (Avandia®), is an active ingredient for a commercially available drug employed to treat diabetes mellitus in a host, e.g. a human being. The typical daily effective dose for the oral administration to a mammal, e.g. a human being, ranges from 2 mg to 8 mg, given as a single dose.

Heretofore, the metformin and rosiglitazone hydrochloride have not been administered together to try to improve the effectiveness of either drug, although co-administration of the two has been proposed *[*Whitcomb; et al., United States Patent No. 6,011,049*].* However, a combined form of the drugs, i.e. a single integral unit thereof has not heretofore been reported. The present invention provides such a single integral unit in the form of a core formulation.

As indicated above, the relative concentrations of each drug is such that a first layer comprising rosiglitazone is prepared. The first layer covers at least a portion of a core comprising metformin. Depending upon the rate of administration and metabolism of the patient destined to be treated, and the concentrations of each ingredient desired for each drug, the first layer may cover only a portion of the core or encompass the entire core. For example, one quarter of the core to about three fourths of the tablet core. The first layer should comprise rosiglitazone, e.g. its hydrochloride, because its dose requirement is lower compared to metformin. Additionally, it is slightly non-polar, its solubility rate slower, and its absorption rate thus dependent on its dissolution rate in the contents of the gastrointestinal tract compared with metformin.

It is to be understood, depending upon the rate of administration to the patient desired, either the first layer or the core may contain a mixture of the two active ingredients or both the first layer and the core may contain the two active ingredients with different and varying concentrations of one or both active ingredients.

The first layer of the core comprises rosiglitazone, e.g. its hydrochloride, in an amount of 0.01% to 20% of the total weight of the core formulation, whereas, the metformin in the core is present in an amount of 10% to 97.5% of the total weight of the core formulation.

Where combinations of the two active ingrdients are present in the first layer and/or the core, the amounts of rosiglitazone, e.g. its hydrochloride, ranges from 1 mg to 45 mg whereas the metformin ranges from 100 mg to 2550 mg.

Finally, it is to be understood that a third pharmacologically active material, e.g. a drug, such as for example sulfonylureas, α-glucosidase inhibitors, meglitinides, and ACE inhibitors may be admixed with the active ingredients in the first layer and/or the core.

The alpha.-glucosidase inhibitors [Jean-Bernard Ducep et al., US Patent No. 5,504,078], bisglucosylmoranoline derivatives [UK Patent No. GB 2 088 365 A], and glucosylmoranoline derivatives [European Patent No. 87112480.6] include the following medicaments: 1.5-Dideoxy-4-O(.alpha.,D-glucopyranosyl)-1,5-[6,7-dideoxy-7-D-glucoheptopyranosyl)imino]-D-glucitol; 1.5-Dideoxy-4-O(.alpha.,D-glucopyranosyl)-1,5-[(1-deoxy-D-fructofuranosyl)imino]-D-glucitol; 1.5-Dideoxy-4-O(.alpha.,D-glucopyranosyl)-1,5-[(4-deoxy-4-D-glucopyranosyl)imino]-D-glucitol; 1.5-Dideoxy-4-O(.alpha.,D-glucopyranosyl)-N-[6-deoxy-1-(6-O-D-glucopyranosyl)-.alpha.-D-glucopyranosyl]-1,5-imino-D-glucitol; 1.5-Dideoxy-4-O(.alpha.,D-glucopyranosyl)-N-[6,7-dideoxy-1-(6-O-D-glucopyranosyl)-7-.alpha.-D -glucoheptopyranosyl]-1,5-imino-D-glucitol; 1.5-Dideoxy-4-O(.alpha.,D-glucopyranosyl)-1,5-[(4-deoxy-4-D-glucopyranosyl)methylimino]-D-glucitol; 1.5-Dideoxy-4-O(.alpha.,D-glucopyranosyl)-N-[4-deoxy-1-(4-O-D-glucopyranosyl)-.alpha.-D-glucopyranosyl]-1,5-imino-D-glucitol; 1.5-Dideoxy-4-O(.alpha.,D-glucopyranosyl)-1,5-{[2(1-D-arabinofuranose)ethyl]imino}-D-glucitol; 1.5-Dideoxy-4-O(.alpha.,D-glucopyranosyl)-N-[4-deoxy-1-(6-O-D-glucopyranosyl)-.alpha.-D-glucopyranosyl]-1,5-imino-D-glucitol; 1.5-Dideoxy-4-O(.alpha.,D-glucopyranosyl)-N-{[4-deoxy-1-(4-O-D-glucopyranosyl)-4-.alpha.-D -glucopyranosyl]methyl}-1,5-imino-D-glucitol; 1.5-Dideoxy-4-O(.alpha.,D-glucopyranosyl)-N-{ [4-deoxy-1-(6-O-D-glucopyranosyl)-4-.alpha.-D -glucopyranosyl]methyl}-1,5-imino-D-glucitol; 1.5-Dideoxy-4-0(.alpha.,D-glucopyranosyl)- 1,5-[(6-deoxy-1-O-methyl-6-β-D-glucopyranosyl)-imino-D -glucitol; 1.5-Dideoxy-4-O(.alpha.,D-glucopyranosyl)-1,5-[(6,7-dideoxy-1-O-methyl-7-13-D-glucoheptopyranosyl) imino]-D-glucitol; 1.5-Dideoxy-4-0(.alpha.,D-glucopyranosyl)-1 ,5-[(1-deoxy-2-O-methyl-β-D-fructofuranosyl)imino]-D-glucitol, 1.5-Dideoxy-4-O(.alpha.,D-glucopyranosyl)-1,5-[(4-deoxy-1-O-methyl-4-β-D-glucopyranosyl)imino]-D-glucitol; 1.5-Dideoxy-4-O(.alpha.,D-glucopyranosyl)-N-[6-deoxy-1-( 1-O-methyl-6-O-β-D-glucopyranosyl)-.alpha.-D -glucopyranosyl]-1,5-imino-D-glucitol; 1.5-Dideoxy-4-O(.alpha.,D-glucopyranosyl)-N-[6,7-dideoxy-1-(1-O-methyl-6-0-β-D-glucopyranosyl)-7-.alpha.-D-glucoheptopyranosyl]-1,5-imino-D-glucitol; 1.5-Dideoxy-4-O(.alpha.,D-glucopyranosyl)-1, 5-[(4-deoxy-1-O-methyl-4-β-D-glucopyranosyl)methylimino]-D-glucitol; 1.5-Dideoxy-4-O(.alpha.,D-glucopyranosyl)-N-[4-deoxy-1-(1-O-methyl-4-O-8-D-glucopyranosyl)-.alpha.-D -glucopyranosyl]-1,5-imino-D-glucitol; 1.5-Dideoxy-4-O(.alpha.,D-glucopyranosyl)-1,5-{[2-(1-O-methyl-1-β-D-arabinofuranosyl)ethyl]imino}-D-glucitol; 1.5-Dideoxy-4-O(.alpha.,D-glucopyranosyl)-N-[4-deoxy-1-(1-O-methyl-6-O-β-D-glucopyranosyl)-.alpha.-D-glucopyranosyl]-1,5-imino-D-glucitol; 1.5-Dideoxy-4-O(.alpha.,D-glucopyranosyl)-N-{[4-deoxy-1-(1-O-methyl-4-O-β-D-glucopyranosyl)-4-.alpha.-D-glucopyranosyl]methyl}-1,5-imino-D-glucitol; 1.5-Dideoxy-4-O(.alpha.,D-glucopyranosyl)-N-{[4-deoxy-1-(1-O-methyl-6-O-β-D-glucopyranosyl)-4-.alpha.-D-glucopyranosyl]methyl}-1,5-imino-D-glucitol; 1.5-Dideoxy-6-O(.alpha.,D-glucopyranosyl)-1,5-[6,7-dideoxy-7-D-glucoheptopyranosyl)imino]-D-glucitol; 1.5-Dideoxy-6-O(.alpha.,D-glucopyranosyl)-1,5-[(1-deoxy-D-fructofuranosyl)imino]-D-glucitol; 1.5-Dideoxy-6-O(.alpha.,D-glucopyranosyl)-1,5-[(4-deoxy-4-D-glucopyranosyl)imino]-D-glucitol; 1.5-Dideoxy-6-O(.alpha.,D-glucopyranosyl)-N-[6-deoxy-1-(6-O-D-glucopyranosyl)-.alpha.-D-glucopyranosyl]-1,5-imino-D-glucitol; 1.5-Dideoxy-6-O(.alpha.,D-glucopyranosyl)-N-[6,7-dideoxy-1-(6-O-D-glucopyranosyl)-7-.alpha.-D -glucoheptopyranosyl]-1,5-imino-D-glucitol;1.5-Dideoxy-6-O(.alpha.,D-glucopyranosyl)-1,5-[(4-deoxy-4-D-glucopyranosyl)methylimino]-D-glucitol; 1.5-Dideoxy-6-O(.alpha.,D-glucopyranosyl)-N-[4-deoxy-1-(4-O-D-glucopyranosyl)-.alpha.-D-glucopyranosyl]-1,5-imino-D-glucitol; 1.5-Dideoxy-6-O(.alpha.,D-glucopyranosyl)-1,5-{[2(1-D-arabinofuranose)ethyl]imino}-D-glucitol; 1.5-Dideoxy-6-0(.alpha.,D-glucopyranosyl)-N-[4-deoxy-1-(6-O-D-glucopyranosyl)-.alpha.-D-glucopyranosyl]-1,5-imino-D-glucitol; 1.5-Dideoxy-6-O(.alpha.,D-glucopyranosyl)-N-{[4-deoxy-1-(4-O-D-glucopyranosyl)-4-.alpha.-D -glucopyranosyl]methyl}-1,5-imino-D-glucitol; 1.5-Dideoxy-6-O(.alpha.,D-glucopyranosyl)-N-{[4-deoxy-1-(6-O-D-glucopyranosyl)-4-.alpha.-D -glucopyranosyl]methyl}-1,5-imino-D-glucitol; 1.5-Dideoxy-6-O(.alpha.,D-glucopyranosyl)-1,5-[(6-deoxy-1-O-methyl-6-β-D-glucopyranosyl) -imino-D-glucitol; 1.5-Dideoxy-6-O(.alpha.,D-glucopyranosyl)-1,5-[(6,7-dideoxy-1-O-methyl-7-β-D-glucoheptopyranosyl) imino]-D-glucitol; 1.5-Dideoxy-6-O(.alpha,D-glucopyranosyl)-1,5-[(1-deoxy-2-O-methyl-13-D-fructofuranosyl)imino]-D-glucitol; 1.5-Dideoxy-6-O(.alpha.,D-glucopyranosyl)-1,5-[(4-deoxy-1-O-methyl-4-β-D-glucopyranosyl)imino]-D-glucitol; 1.5-Dideoxy-6-O(.alpha.,D-glucopyranosyl)-N-[6-deoxy-1-(1-O-methyl-6-O-β-D-glucopyranosyl)-.alpha.-D -glucopyranosyl]-1,5-imino-D-glucitol; 1.5-Dideoxy-6-O(.alpha.,D-glucopyranosyl)-N-[6,7-dideoxy-1-(1-O-methyl-6-O-β-D-glucopyranosyl) -7-.alpha.-D-glucoheptopyranosyl]-1 ,5-imino-D-glucitol; 1.5-Dideoxy-6-O(.alpha.,D-glucopyranosyl)-1,5-[(4-deoxy-1-O-methyl-4-β-D-glucopyranosyl)methylimino]-D-glucitol; 1.5-Dideoxy-6-O(.alpha.,D-glucopyranosyl)-N-[4-deoxy-1-(1-O-methyl-4-O-β-D-glucopyranosyl)-.alpha.-D -glucopyranosyl]-1,5-imino-D-glucitol; 1.5-Dideoxy-6-O(.alpha.,D-glucopyranosyl)-1,5-{[2-(1-O-methyl-1-B-D-arabinofuranosyl)ethyl]imino}-D-glucitol; 1.5-Dideoxy-6-O(.alpha.,D-glucopyranosyl)-N-[4-deoxy-1-(1-O-methyl-6-O-β-D-glucopyranosyl)-.alpha.-D - glucopyranosyl]-1,5-imino-D-glucitol; 1.5-Dideoxy-6-O(.alpha.,D-glucopyranosyl)-N-([4-deoxy-1-(1-O-methyl-4-O-B-D-glucopyranosyl)-4-.alpha.-D-glucopyranosyl]methyl}-1,5-imino-D-glucitol; 1.5-Dideoxy-6-0(.alpha.,D-glucopyranosyl)-N-([4-deoxy-1-(1-O-methyl-6-O-β-D-glucopyranosyl)-4-.alpha.-D-glucopyranosyl]methyl}-1,5-imino-D-glucitol.

The list of medicaments includes acid addition salt forms with such inorganic acids such as, for example, hydrochloric, hydrobromic, sulfuric, phosphoric and like acids; with organic carboxylic acids such as, for example, acetic, propionic, glycolic, lactic, pyruvic, malonic, succinic, fumaric, maleic, tartaric, citric, ascorbic, maleic, hydroxymaleic and dihydroxymaleic, benzoic, 2-acetoxybenzoic, mandelic and like acids; and with organic sulfonic acids such as methanesulfonic acid and p-toluenesulfonic acid.

The sulfonylureas are a class of compounds that have been widely employed to treat diabetes. Such compounds are well known, for example, as described in U.S. Pat. Nos. 3,454,635; 3,669,966; 2,968,158; 3,501,495; 3,708,486; 3,668,215; 3,654,357; and 3,097,242. Especially preferred sulfonylureas to be employed in the combinations of this invention are glyburide, gliquidone, glipizide, tolbutamide, tolazamide, glisoxepid, chlorpropamide, glibornuride, gliclazide, glimepiride, phenbutamide, and tolcyclamide. Other medicaments, for example an antibiotic, a vitamin, a drug that works on the heart, or in the liver, may be admixed with the active ingredients in the first layer and/or the core.

The resultant core having the first layer thereon is prepared by any conventional means known in the pharmaceutical art, e.g. compression, tabletting technology, spraying technology, or encapsulation in a pharmaceutically acceptable presentation, such as a gelatin capsule. In particular, typically the core formulation of the present invention is preferably fabricated by compression into a tablet.

The resultant core formulation of the present invention is useful to treat diabetes mellitus. Surprisingly the resultant core formulation of the invention is as user friendly and clinically effective as compared to the administration of metformin alone or rosiglitazone, e.g. its hydrochloride, as demonstrated by co-administration of the two agents *[*Whitcomb; et al., United States Patent No. 6,011,049*],* where in general, the incidence of adverse events was not influenced by age or menopausal status, and further, patients treated with the combination therapy attained better glycemic control than with either monotherapy.

It is to be understood, however, that for any particular subject being treated, e.g., a mammal, specific dosage regimens should be adjusted according to the individual need. It is further to be understood that the dosages set forth herein are exemplary only and that they do not, to any extent limit the scope of the practice of the present invention.

The core formulation of the present invention may be administered orally, for example, with inert diluent or with an edible carrier. For the purpose of oral therapeutic administration, the core formulation may have excipients incorporated therein. The subject core formulation may also contain the following adjuvants: a binder such as microcrystalline cellulose, gum tragacanth or gelatin; an excipient such as starch or lactose, a disintegrating agent such as alginic acid, Primogel®, corn starch and the like; a lubricant such as magnesium stearate or Sterotex; a glidant such as colloidal silicon dioxide; and a sweetening agent such as sucrose or saccharin may be added or a flavoring agent such as peppermint, methyl salicylate or orange flavoring.

The subject core formulation of the invention may contain other various materials which modify the physical form of the dosage unit (the subject core formulation), for example, as coatings. Thus, the subject core formulation of the present invention may be coated with sugar, shellac or other enteric coating agents. Materials used in preparing these various compositions should be pharmaceutically pure and non-toxic in the amounts used.

In an alternative embodiment of the present invention. The resultant core formulation (having a first layer completely or partially covering the core), is treated whereby an outer shell, at least a portion of which comprises a biodegradable material having a predetermined rate of degradation or metabolism in the host being treated, is formed which encloses the particles of the first layer and/or the core.

The biodegradable material is a high molecular weight compound, which is physiologically acceptable and decomposes in the body of the human being or other animal and is absorbed.

The biodegradable material, comprising the outer shell, having a predetermined rate of degradation or metabolism or break down, is selected from those materials well known in the art, which do not react with metformin and/or rosiglitazone, e.g. its maleate, hydrochloride. Such materials include, biodegradable polymers, such as polyorthoesters, polyanhydrides, polyamides based on glutamic acid, polyalkyl cyanoacrylates, polyesters of lactic and glycolic acid, polyactide polymers, cellulosic polymers, polyvinyl acetate, polyvinyl alcohol, polyvinylchloride, natural and synthetic rubbers, polyacrylates, polystyrene, etc. Additionally, reference is made to U.S. Patents Nos. 4,166,800, and 4,389,330, which disclose additional shell forming materials.

The shell encapsulating the particles of rosiglitazone, e.g. its hydrochloride, of the first layer and/or the particles of metformin of the core is obtained by any conventional microencapsulation process whereby microspheres of metformin and/or rosiglitazone, e.g. its hydrochloride, are formed, e.g. a solvent removal process, a phase separation technique, coacervation etc. In this regard reference is made to U.S. Patents Nos. 4,166,800 and 4,389,330, Conte et al, J. Controlled Release, vol. 26, (1993), pages 39-47; and U.S. Patent No. 4,839,177.

In a variation of the above alternative embodiment, the resultant core formulation is treated whereby only the top surface area of the first layer has a shell coating thereon. In this regard, reference is made to U.S. Patent No. 5,916,584, which describes the process for forming such a shell. The resulting core formulation having the first layer encapsulated by the shell comprising the shell material, is one which provides a delay time prior to release of the active ingredients, i.e. for example, rosiglitazone hydrochloride and metformin, to the patient being treated for diabetes mellitus.

It is to be understood that for either metformin or rosiglitazone, any pharmaceutically acceptable form may be used, such as for example the free acids, free bases, salts and various hydrate forms, including semi-hydrate forms, of these medicaments, as well as other pharmaceutical materials which are used in the formulation process as acceptable excipient materials generally known to those skilled in the art.

It is understood that any one of the biguanides, i.e. drugs having the action of the stimulation of anaerobic glycolysis, is covered by this invention as these, like metformin, increase the sensitivity to insulin in peripheral tissues of the host, e.g. a human being or another animal. These compounds also are involved in the inhibition of glucose absorption from the intestine, suppression of hepatic gluconeogenesis, and inhibition of fatty acid oxidation. Examples of other typical biguanides included in this application are phenformin, buformin etc.

## Claims

1. A core formulation comprising:
(a) a first layer comprising rosiglitazone or a pharmaceutically acceptable salt thereof; and
(b) a core, at least a portion of which is enclosed by said first layer, comprising a biguanide; and
(c) wherein said rosiglitazone, said biguanide or both are in microsphere form.

2. The formulation as defined in claim 1, wherein said rosiglitazone is present in an amount ranging from about 1 mg to about 15 mg and said biguanide is metformin present in an amount ranging from about 10 mg to about 4000 mg.

3. The formulation as defined in claim 2 which further comprises:
(a) a biodegradable shell consisting of biodegradable polymers, polyamides based on glutamic acid, polyalkyl, cyanoacrylates, polyesters of lactic acid and glycolic acid, polyactide polymers, cellulosic polymers, polyvinyl acetate, polyvinyl alcohol, polyvinylchloride, natural and synthetic rubbers, polyacrylates, or polystyrene or mixtures thereof of any of the above; and
(b) wherein said biodegradable shell (i) covers at least a portion of said first layer and (ii) is for delaying the release of said rosiglitazone.

4. The formulation as defined in claim 1 wherein said core comprises metformin or a pharmaceutically acceptable salt thereof.

5. A method for producing the formulation as defined in clam 3, which comprises:
(a) producing a hollow outer shell comprising a biodegradable shell consisting of biodegradable polymers, polyamides based on glutamic acid, polyalkyl cyanoacrylates, polyesters of lactic acid and glycolic acid, polyactidepolymers, cellulosic polymers, polyvinyl acetate, polyvinyl alcohol, polyvinylchloride, natural and synthetic rubbers, polyacrylates, or polystyrene or any mixtures thereof;
(b) inserting a core, comprising metformin and having an outer layer comprising rosiglitazone or a salt thereof partially enclosing said core, into said hollow outer shell wherein said rosiglitazone, said biguanide or both are in microsphere form; and
(c) sealing said core within said hollow outer shell.

6. A method for producing the formulation as defined in claim 4, which comprises:
(a) forming a core of said metformin; and
(b) depositing a layer of said rosiglitazone on at least a portion of a surface of said core.

7. A formulation according to any one of claims 1 to 4 for use in treating diabetes mellitus in a patient in need thereof, wherein said active ingredients are each present in an effective amount.

8. A formulation according to claim 1 for use in treating diabetes mellitus in a patient in need thereof wherein the biguanide is phenformin.

9. A formulation according to claim 1 for use in treating diabetes mellitus in a patient in need thereof; wherein the biguanide is buformin.

## Patentansprüche

1. Kernformulierung, umfassen:
(a) eine erste Schicht, umfassend Rosiglitazon oder ein pharmazeutisch verträgliches Salz davon; und
(b) einen Kern, von dem mindestens ein Teil durch die erste Schicht eingeschlossen ist, umfassend Biguanid; und
(c) wobei Rosiglitazon, Biguanid oder beide in Form von Mikrokügelchen vorliegen.

2. Formulierung nach Anspruch 1, wobei das Rosiglitazon in einer Menge vorliegt, die im Bereich von etwa 1 mg bis etwa 15 mg liegt, und das Biguanid Metformin ist, das in einer Menge vorliegt, die im Bereich von etwa 10 mg bis etwa 4000 mg liegt.

3. Formulierung nach Anspruch 2, welche ferner umfasst:
(a) eine biologisch abbaubare Hülle, bestehend aus biologisch abbaubaren Polymeren, Polyamiden auf Basis von Glutaminsäure, Polyalkylcyanoacrylaten, Polyestern von Milchsäure und Glykolsäure, Polyactidpolymeren, Cellulosepolymeren, Polyvinylacetat, Polyvinylalkohol, Polyvinylchlorid, natürlichen und synthetischen Kautschuken, Polyacrylaten oder Polystrol oder Gemischen davon von einem der Vorstehenden; und
(b) wobei die biologisch abbaubare Hülle (i) mindestens einen Teil der ersten Schicht bedeckt und (ii) zum Verzögern der Freisetzung des Rosiglitazons vorliegt.

4. Formulierung nach Anspruch 1, wobei der Kern Metformin oder ein pharmazeutisch verträgliches Salz davon umfasst.

5. Verfahren zur Herstellung der Formulierung nach Anspruch 3, welches umfasst:
(a) Herstellen einer hohlen Außenhülle, umfassend eine biologisch abbaubare Hülle, bestehend aus biologisch abbaubaren Polymeren, Polyamiden auf Basis von Glutaminsäure, Polyalkylcyanoacrylaten, Polyestern von Milchsäure und Glykolsäure, Polyactidpolymeren, Cellulosepolymeren, Polyvinylacetat, Polyvinylalkohol, Polyvinylchlorid, natürlichen und synthetischen Kautschuken, Polyacrylaten oder Polystrol oder jedem Gemisch davon;
(b) Einfügen eines Kerns, umfassend Metformin, und der eine Außenschicht aufweist, umfassend Rosiglitazon oder ein Salz davon, die den Kern teilweise einschließt, in die hohle Außenhülle, wobei das Rosiglitazon, das Biguanid oder beide in Form von Mikrokügelchen vorliegen; und
(c) Versiegeln des Kerns im Inneren der hohlen Außenhülle.

6. Verfahren zur Herstellung der Formulierung nach Anspruch 4, welches umfasst:
(a) (a) Erzeugen eines Kernes des Metformins; und
(b) Auftragen einer Schicht des Rosiglitazons auf mindestens einen Teil einer Oberfläche des Kerns.

7. Formulierung nach einem der Ansprüche 1 bis 4 zur Verwendung bei der Behandlung von Diabetes mellitus bei einem Patienten mit einem Bedarf dafür, wobei die Wirkstoffe jeweils in einer wirksamen Menge vorliegen.

8. Formulierung nach Anspruch 1 zur Verwendung bei der Behandlung von Diabetes mellitus bei einem Patienten mit einem Bedarf dafür, wobei das Biguanid Phenformin ist.

9. Formulierung nach Anspruch 1 zur Verwendung bei der Behandlung von Diabetes mellitus bei einem Patienten mit einem Bedarf dafür, wobei das Biguanid Buformin ist.

## Revendications

1. Formulation de noyau comprenant:
(a) une première couche comprenant la rosiglitazone ou un sel pharmaceutiquement acceptable de celui-ci ; et
(b) un noyau, dont au moins une partie de celui-ci est enveloppée par ladite première couche, comprenant un biguanide ; et
(c) dans laquelle ladite rosiglitazone, ledit biguanide ou les deux sont sous forme de microsphère.

2. Formulation selon la revendication 1, dans laquelle ladite rosiglitazone est présente en quantité variant d'environ 1 mg à environ 15 mg et ledit biguanide est la metformine qui est présente en quantité variant d'environ 10 mg à environ 4000 mg.

3. Formulation selon la revendication 2 comprenant en outre :
(a) une enveloppe biodégradable constituée de polymères biodégradables, de polyamides à base d'acide glutamique, de poly(cyanoacrylates d'alkyle), de polyesters d'acide lactique et d'acide glycolique, de polymères polyactides, de polymères cellulosiques, de poly(acétate de vinyle), de poly(alcool vinylique), de poly(chlorure de vinyle), de caoutchoucs naturels et synthétiques, de polyacrylates, ou de polystyrène, ou de mélanges des quelconques constituants précités ; et
(b) dans laquelle ladite enveloppe biodégradable (i) recouvre au moins une partie de ladite première couche et (ii) est destinée à retarder la libération de ladite rosiglitazone.

4. Formulation selon la revendication 1, dans laquelle ledit noyau comprend de la metformine ou un sel pharmaceutiquement acceptable de celle-ci.

5. Procédé de production de la formulation selon la revendication 3, comprenant :
(a) la production d'une enveloppe externe creuse comprenant une enveloppe biodégradable constituée de polymères biodégradables, de polyamides à base d'acide glutamique, de poly(cyanoacrylates d'alkyle), de polyesters d'acide lactique et d'acide glycolique, de polymères polyactides, de polymères cellulosiques, de poly(acétate de vinyle), de poly(alcool vinylique), de poly(chlorure de vinyle), de caoutchoucs naturels et synthétiques, de polyacrylates ou de polystyrène ou de leurs mélanges ;
(b) l'insertion d'un noyau, comprenant la metformine et ayant une couche externe comprenant la rosiglitazone ou un de ses sels, enfermant partiellement ledit noyau, dans ladite enveloppe externe creuse, ladite rosiglitazone, ledit biguanide ou les deux étant sous forme de microsphère ; et
(c) le scellage dudit noyau à l'intérieur de ladite enveloppe externe creuse.

6. Procédé pour produire la formulation selon la revendication 4, comprenant :
(a) la formation d'un noyau constitué de ladite metformine ; et
(b) le dépôt d'une couche de ladite rosiglitazone sur au moins une partie d'une surface dudit noyau.

7. Formulation selon l'une quelconque des revendications 1 à 4, pour un usage dans le traitement du diabète sucré chez un patient qui nécessite ce traitement, dans laquelle lesdits ingrédients actifs sont présents, chacun, en quantité efficace.

8. Formulation selon la revendication 1 pour un usage dans le traitement du diabète sucré chez un patient qui nécessite ce traitement, dans laquelle le biguanide est la phenformine.

9. Formulation selon la revendication 1 pour un usage dans le traitement du diabète sucré chez un patient qui nécessite ce traitement, dans laquelle le biguanide est la buformine.
